# EUROPEAN PATENT SPECIFICATION

(11) **EP 1 214 084 B1**
(45) Date of publication and mention of the grant of the patent: **06.04.2005**
(21) Application number: 00962472.7
(22) Date of filing: 08.09.2000
(51) Int. Cl.: A61K 35/78, A61P 9/00

(54) **AN ANTI-OXIDANT PREPARATION BASED ON PLANT EXTRACTS FOR THE TREATMENT OF CIRCULATION AND ADIPOSITY PROBLEMS**
EIN ANTIOXYRIERUNGSMITTEL AUF BASIS VON PFLANZENEXTRAKTEN ZUR BEHANDLUNG VON KREISLAUFS- UND FETTSUCHTPROBLEMEN
PREPARATION ANTIOXYDANTE A BASE D'EXTRAITS DE PLANTES PERMETTANT DE TRAITER DES PROBLEMES DE CIRCULATION ET D'ADIPOSITE

(30) Priority: 10.09.1999 CH 166399
(43) Date of publication of application: 19.06.2002
(73) Proprietor: Ceteris Holding B.V.-Amsterdam (Olanda) - Succursale di Lugano, 6900 Lugano (CH)
(72) Inventor: MERIZZI, Gianfranco, I-10128 Torino (IT)
(74) Representative: Rambelli, Paolo
(86) International application number: PCT/EP2000/008875
(87) International publication number: WO 2001/019158

(56) References cited:
- GB-A- 2 174 904
- W.REILLY, V.REEVE: "Body contouring using an oral herbal antioxidant formulation-Centelaplus: a dose controlled observational study" REDOX REPORT, vol. 5, no. 2-3, 2000, pages 144-145, XP000990069

## Description

The present invention relates to a preparation based on plant extracts which has an anti-oxidant effect and is particularly useful in the treatment of circulation problems such as phlebitis, varicose veins, arteriosclerosis, haemorrhoids and high blood pressure, as well as in the prevention and treatment of surplus fat.

The object of the invention is to provide a preparation to be taken orally, based on a combination of active ingredients of natural and plant origin, which work more effectively to prevent and treat the aforesaid problems when administered by mouth.

This object is achieved according to the invention by providing a preparation characterised in that its active ingredients include a combination of *Ginkgo biloba* biflavones, catechine and/or epicatechine, cumarine and derivatives thereof, iodine and an ingredient chosen from asiaticoside, asiatic acid, madecassic acid and compounds thereof.

The preparation is obtained by mixing plant extracts which contain the above active components.

It is known that extracts from the leaves of *Ginkgo biloba* contain important active components and in particular flavonol glucosides, lactonic terpenes and dimeric biflavones or flavones. The flavonol glucosides and the lactonic terpenes constitute the active components of the standardized *Ginkgo biloba* extracts currently available on the market; however these extracts do not contain the biflavone component which is not extracted during normal processing. The *Ginkgo biloba* extract used in compositions according to the present invention is highly enriched with the biflavone component and, as a possible option, with extracts containing flavonol glucosides and lactonic terpenes. Five biflavones in particular have been identified in the biflavone component of *Ginkgo biloba*: these are, in particular, amentoflavone, bilobetine, isoginkgetine, ginkgetine and sciadopisitine; the five said compounds differ only by the presence of methyl groups in some positions and, like all flavones, are powerful antioxidants. However, from a pharmacological point of view, they are characterised by their anti-phosphodiesterase, anti-inflammatory, vasculokinetic and anti-allergy properties. Phosphodiesterases (PDE) are cellular enzymes responsible for interacting with cyclic nucleotides so as to linearize them. Cyclic nucleotides are involved as second messengers in transmitting intercellular signals and are thus responsible for some phenomena which are very important from a biochemical point of view. They assist with the visual process and in the relaxation of smooth muscles, they stimulate lipolysis in adiposity and vasculo-motion in capillary arterioles. More specifically, it is sufficient to report that in inhibiting PDE depending on cyclic AMP, these biflavones demonstrate an IC50 of 1.2 micromoles.

The anti-inflammatory properties of biflavones, and in particular those of amentoflavone, have been demonstrated both in vitro, by measuring the interaction of these biflavones with cyclo-oxygenase, lipo-oxygenase and phospholipase A2, and in vivo, using various models of inflammation in animals (carragineen oedema, Croton oil inflammation etc). The anti-inflammatory action of the biflavones was confirmed both in models using local application and in those in which they were administered peritoneally. In these models, the biflavones always demonstrated an anti-inflammatory action equivalent to that of indomethacyn or prednisolone. This effectiveness can be explained by analysing the IC50 of cycol-oxygenase inhibition, which for amentoflavone is 3 micromoles.

With regard to the microvasculokinetic activity of biflavones, it should be reported that, following acute treatment, these substances improve the size of the arterial sphygmic wave and, following chronic treatment they improve capillary density in tissues with trophic-connective problems, such as those affected by panniculopathy and/or various degrees of sclerodermy. Biflavones also have clear anti-allergy properties; they inhibit the release of histamine by mast-cells stimulated by allergens.

In the context of the present invention, it has been demonstrated that, when administered orally, the activity of the aforesaid biflavones, possibly in combination with flavonol glucosides and lactonic terpenes which are normally present in standard *Ginkgo biloba* extracts, is enhanced when the latter are combined with the aforesaid active compounds.

The extracts are preferably used in a phytosomal form, in which the active components are compounded with phospholipids.

In the context of the invention it is convenient to use an extract of leucocyanidine or leucoanthocyanine derived from *Vitis vinifera* as the source of catechine or epicatechine. Leucoanthocyanines are procyanidolic oligomers derived from condensing monomeric units of flavan-3-ols and flavan-3,4-diols, these being either free or esterified with gallic acid. Leucocyanines are powerful anti-oxidant substances with the ability to protect capillaries by increasing oxygen to the tissue; these active substances prove biologically active even when administered orally and they have been shown to be tropic for the cardio-vascular system and for all tissues, such as artery walls, which are rich in glucoaminoglycene. Preferably, phytosomal forms of extracts are used, thus further enhancing the bioavailability of the active principles. In this form the procyanodines are completed with phospholipids, particularly distearylphosphatidycoline of soya.

The preferable source of cumarine is an extract of Melilotus (Melilotus officinalis), cumarine and its derivatives being the main active ingredients thereof; the main active ingredients of this extract are melilotine (3,4 dihydro-cumarine), melilotic acid (hydroxycumarinic acid), melilotoxide (a melilotine glucoside) and some flavinoids which act like vitamin P; the active ingredients contained in the extract are particularly effective in increasing capillary strength, in reducing vascular permeability, in stimulating venous circulation and improving lymphatic circulation.

Extract of Melilotus may be replaced or backed up, as a source of cumarine and its derivatives, by an extract of *Aesculus hippocastanum* (horse chestnut) in the same dosage or up to around twice the dose of Melilotus extract.

The most abundant active principle of *Aesculus hippocastanum* extract, obtained from the bark, the pericarp of the fruit, the leaves or the buds, is cumarine glucoside, esculoside (6-0-glucosil-7-hydroxy-cumarine). other cumarines contained in the extract are fraxine (8-0-glycoside-7-hydroxy-6-metoxycumarine) and the aglicones, esculetin (6,7-dioxy-cumarine) and fraxetine (7,8-dioxy-6-methoxy-cumarine).

The preferred source of asiaticoside, asiatic acid and madecassic acid is an extract containing a triterpenic fraction of centella (*Centella Asiatica*) which contains a combination of the above three active principles. The extract should preferably be used in a phytosomal form, obtained by a reaction between the triterpenic fraction of *Centella Asiatica* with a phospholipid. A main action of the triterpenic fraction of centella consists in accelerating the uptake and metabolism of lysine and of proline, thus increasing the synthesis and the release of tropocollagen and stimulating the turnover of acid mucopolysaccharide acids in connective tissue. Thanks to these properties, the active principles are particularly effective in reducing localised adiposity.

The preferred source of iodine is an extract of *Fucus vesiculosus,* a seaweed of the Fucaceae family. The role of the iodine in *Fucus vesiculosus* is currently well characterised; it is made more readily available by complexing with a protein fraction of the extract; it increases the synthesis of thyroid hormones and indirectly enhances the lipolitic action of these hormones (T3 and T4) thanks to a local thermogenic action on adipose tissue. The Fucus extract also contains polysaccharides such as fucoidin, alginic acid, laminarin and poliose; of these, the alginic acids, in particular, enhance the effect of the extract since they are hydrophilic molecules able to swell from their original volume in the presence of water; thanks to this characteristic, when ingested they not only reduce the appetite but also increase the speed of transit of food through the intestines, thus ensuring that it is less well absorbed.

The basic composition of the invention can thus be obtained by mixing a biflavone extract of *Ginkgo biloba* (perhaps in combination with a standard *Ginkgo biloba* extract also containing flavonol glucosides and lactonic terpenes), leucocyanidine extract, Melilotus extract, Centella extract and extract of *Fucus vesuculosus*; these extracts preferably being in a phytosome form.

With reference to the extracts normally available on the market, the basic composition is preferably made up by the following percentages by weight:
1.5 - 32% biflavone extract of *Ginkgo biloba*;
6 - 80% of leucocyanidine extract;
1.5 - 60%, preferably 1.5 - 32% of melilotus extract and/or *Aesculus hippocastanum* extract;
1.5 - 32% of centella extract;
1.5 - 85% *Fucus vesiculosus* extract, possibly in combination with:
1.5 - 32% of standard *Ginkgo biloba* extract containing flavon glucosides and lactonic terpenes.

In terms of the content of active principles, the composition of the invention preferably contains the following percentages by weight:
0.2 - 12%, preferably 0.5 - 6% of total biflavones, expressed as ginkgetine content;
0.2 - 12%, preferably 0.5 - 8% of catechine and/or epicatechine, expressed as catechine content;
0.15 - 7%, preferably 0.3 - 3.5% of cumarine and its derivatives;
0.25 - 15%, preferably 0.5 - 7.5% of asiaticoside;
0.25 - 22%, preferably 0.5 - 11 % of asiatic acid and/or madecassic acid;
up to 0.5% of iodine and possibly one or more of its components:
0.25 - 12%, preferably 0.5 - 6%, of flavonol glucosides, and
0.1 - 2%, preferably 0.2 - 1% of Ginkolide lactonic terpenes (bilobalide).

The composition can also contain active ingredients chosen from methylxanthinic derivatives, -such as caffeine, theobromine or theophylline in particular; mono and di-caffeoylchinic acids, chlorogenic acids, eicosapentaenoic acid (EPA), docahexaenoic acid (DHA) gamma-linolenic acid and combinations thereof.

The preferred source of methylxanthinic derivatives, of caffeine and of chlorogenic acids in particular, is an extract of *Ilex paraguariensis*, possibly in phytosomal form; the standard, commercially available extract can be added to the previously-described basic mixture in a quantity of 1.5 to 32% by weight.

Caffeoylchinic acid and its derivatives (such as cynarine) are preferably introduced by means of an artichoke extract (*Cynara scolymus*); this extract typically also contains caffeic acid and chlorogenic acid; this extract is preferably used in quantities of 1.5 to 32% by weight with reference to 100 parts of the basic mixture.

The preferable source of eicosapentaenoic acid (EPA) and of docohexaenoic acid (DHA) is fish oil which, with reference to 100 parts of the basic mixture, may be added in quantities of 5 to 80% by weight.

Gamma-linolenic acid is preferably introduced into the formulation by the use of borage oil, added in quantities of 30 to 120% by weight with reference to 100 parts of basic preparation.

In particular, in the preferred embodiment of the invention, the composition includes one or more of the following components:
Caffeine in quantities of 0.05 to 2.5 (preferably 0.1 - 1%) by weight with reference to the total preparation,
Caffeoylchinic acids in quantities from 0.05 to 4.8% (preferably 0.01 to 2.5%) by weight,
Eicosapentanoic acid in quantities from 0.75 to 24% (preferably from 1.5 to 12%) by weight,
Docohexanoic acid in quantities from 0.6 to 8% (preferably 1.2 to 4%) by weight,
Gamma-linolenic acid in quantities from 2.5 to 22%, preferably 5-11% by weight.

For example, a typical composition could be formulated according to the data in the table below, which gives the preferred minimum and maximum quantities expressed in parts by weight of the components of the basic mixture (marked with an asterisk) and of optional ingredients.

| | **Minimum** (Parts by weight) | **Maximum** (Parts by weight) |
|---|---|---|
| *Dry extract of *Vitis vinifera* (optionally phytosomes) | 20 | 200 |
| *Dry extract of Melilotus officinalis and/or Aesculus hippocastanum | 5 | 40 |
| **Ginkgo biloba* biflavones (optionally phytosomes) | 5 | 50 |
| Dry extract of *Ginkgo biloba* (optionally phytosomes) | 5 | 50 |
| *Dry extract of *Fucus vesiculosus* | 50 | 200 |
| *Dry extract of *Centella asiatica* (optionally phytosomes) | 10 | 50 |
| Dry extract of artichoke | 10 | 100 |
| Dry extract of *Ilex paraguariensis* | 10 | 100 |
| *Borage oil* | 50 | 1000 |
| *Fish oil* | 50 | 750 |
| Soya lecithin | 20 | 100 |

In the above table, the given values, expressed in parts by weight, correspond, when expressed in milligrams to the minimum and maximum daily doses recommended or to the dose per capsule.

The composition of the invention is formulated in forms suited to be taken orally, such as, for example, gelatin capsules with either soft or rigid shells, tablets, pills, elixirs, suspensions and syrups. The various forms can include excipients and/or binders and/or pharmaceutically acceptable vehicles, in particular lecithin mono- and diglycerides of fatty acids. The mixture of extracts can be administered orally, possibly in an edible vehicle or can be incorporated directly into food as part of a diet.

The preparation is particularly useful in treating localised adiposity, in particular in men but also in women. It is well known that adiposity differs in men and in women, by the area in which it is deposited, by the quantity, by functional response and by consequences for health; in actual fact this surplus fat ("spare tyres" in men and cellulite in women) is due, at least in part, to a tropism which is known to involve both vascular and connective tissue, as well as adispose tissue. The composition of the invention provides an association of well characterised substances from a pharmaco-toxicological and chemical point of view, which is totally free of side effects and is particularly advantageous compared to pharmaceutical preparations based on appetite-suppressant compounds, as used in conventional pharmacological treatment.

## Claims

1. A composition based on plant extracts, with an anti-oxidant activity which is particularly useful in the prevention and treatment of circulation problems and in the prevention and treatment of adiposity, **characterised in that** its active ingredients comprise, in association, biflavones of *Ginkgo biloba*, catechine and/or epicatechine, cumarine or derivatives thereof, iodine and a component chosen from among madecassic acid, asiatic acid, asiaticoside and mixtures of these.

2. A composition according to Claim 1, **characterised in that** it is obtained by mixing plant extracts containing the aforesaid active principles.

3. A composition according to Claim 2, charcaterised in that the said extracts are in phytosomal form.

4. A composition according to any one of the preceding Claims, **characterised in that** it further includes flavonol glucosides and lactonic terpenes.

5. A composition according to any Claim from 1 to 4, **characterised in that** it also includes an active principle chosen from the group consisting of methylxanthinic derivatives, chlorogenic acids, mono- and di-caffeoylchinic acid and derivatives thereof, eicosapentaenoic acid, docohexaenoic acid, gamma-linolenic acid and mixtures thereof.

6. A composition according to any of Claims 1 to 4, **characterised in that** it is obtained by mixing plant extracts in the following percentages by weight:
1.5-32% of *Ginkgo biloba* biflavone extract;
6-80% of leucocyanidine;
1.5-32% of *Melilotus* and/or *Aesculus hyppocastanum* extract;
1.5-32% of centella extract;
1.5-85% extract of *Fucus vesiculosus*; and optionally
1.5-32% of standardised *Ginkgo biloba* extract containing flavone glucosides and lactonic terpenes.

7. A composition according to Claim 6, **characterised in that** with reference to 100 parts of the basic mixture of Claim 6, it also includes one or more of the following components:
from 1.5 to 32% by weight of *Ilex paraguariensis* extract;
from 1.5 to 32% by weight of artichoke extract;
from 5 to 80% by weight of fish oil;
and
from 30 to 120% by weight of borage oil.

8. A composition according to any one of the preceding Claims which includes:
0.12-12%, preferably 0.5-6% by weight, of total biflavones;
0.2-12%, preferably 0.5-8% by weight, of catechine and/or epicatechine;
0.15-7%, preferably 0.3-3.5% by weight, of cumarine and derivatives thereof;
0.25-15%, preferably 0.5-7.5% by weight of asiaticoside;
0.25-22%, preferably 0.5-11% by weight, of asiatic acid and/or madecassic acid;
up to 0.5% of iodine and, optionally
0.25-12%, preferably 0.5-6% by weight, of flavonol glucosides and
0.1-2%, preferably 0,2-1% by weight, of lactonic terpenes.

9. A composition according to Claim 8, **characterised in that** it also includes one or more of the following components:
from 0.05 to 2.5%, preferably 0.1-1% by weight, of caffeine;
from 0.05 to 4.8%, preferably 0.1-2.5% by weight, of caffeilchinic acids;
from 0.75 to 24%, preferably 1.5-12% by weight, of eicosapentaenoic acid;
from 0.6 to 8%, preferably 1.2-4% by weight, of docohexaenoic acid; and
from 2.5 to 22%, preferably from 5 to 11% by weight of gamma-linolenic acid.

10. A composition according to any one of the preceding Claims in a pharmaceutical form for oral administration.

## Patentansprüche

1. Zusammensetzung, beruhend auf Pflanzenextrakten, mit einer antioxidativen Wirksamkeit, die besonders bei der Prävention und Behandlung von Kreislaufproblemen und bei der Prävention und Behandlung von Adipositas nützlich ist, **dadurch gekennzeichnet, daß** ihre Wirkstoffe in Assoziation Biflavone von *Ginkgo biloba*, Catechine und/oder Epicatechine, Cumarine oder Derivate davon, Iod und eine Komponente, gewählt unter Madekassischer Säure, Asiatischer Säure, Asiaticosid und Mischungen von diesen, umfassen.

2. Zusammensetzung nach Anspruch 1, **dadurch gekennzeichnet, daß** sie durch Mischen von Pflanzenextrakten erhalten wird, welche die vorgenannten Wirkprinzipien enthalten.

3. Zusammensetzung nach Anspruch 2, **dadurch gekennzeichnet, daß** die Extrakte in Phytosomform vorliegen.

4. Zusammensetzung nach einem der vorhergehenden Ansprüche, **dadurch gekennzeichnet, daß** sie außerdem Flavonolglucoside und Lactonterpene enthält.

5. Zusammensetzung nach einem der Ansprüche 1 bis 4, **dadurch gekennzeichnet, daß** sie außerdem ein Wirkprinzip enthält, das aus der Gruppe bestehend aus Methylxanthinderivaten, Chlorogensäuren, Mono- und Dicaffeoylchinasäure und Derivaten davon, Eicosapentaensäure, Docohexaensäure, Gamma-Linolensäure und Mischungen davon gewählt ist.

6. Zusammensetzung nach einem der Ansprüche 1 bis 4, **dadurch gekennzeichnet, daß** sie durch Mischen von Pflanzenextrakten in den folgenden prozentualen Gewichtsanteilen erhalten wird:
1,5-32% Biflavon-Extrakt aus *Ginkgo biloba*;
6-80% Leucocyanidin;
1,5-32% *Melilotus-* und/oder *Aesculus*-*hyppocastanum*-Extrakt;
1,5-32% Centella-Extrakt;
1,5-85% Extrakt *aus Fucus vesiculosus*; und wahlweise
1,5-32% standardisierter *Ginkgo*-*biloba*-Extrakt, der Flavonglucoside und Lactonterpene enthält.

7. Zusammensetzung nach Anspruch 6, **dadurch gekennzeichnet, daß** sie bezogen auf 100 Teile der Grundmischung von Anspruch 6 auch eine oder mehrere der folgenden Komponenten enthält:
1,5 bis 32 Gew.-% *Ilex-paraguariensis*-Extrakt;
1,5 bis 32 Gew.-% Artischockenextrakt;
5 bis 80 Gew.-% Fischöl; und
30 bis 120 Gew.-% Borretschöl.

8. Zusammensetzung nach einem der vorhergehenden Ansprüche, welche enthält:
0,12 bis 12 Gew.-%, vorzugsweise 0,5 bis 6 Gew.-%, Gesamtbiflavone;
0,2 bis 12 Gew.-%, vorzugsweise 0,5 bis 8 Gew.-%, Catechin und/oder Epicatechin;
0,15 bis 7 Gew.-%, vorzugsweise 0,3 bis 3,5 Gew.-%, Cumarin und Derivate davon;
0,25 bis 15 Gew.-%, vorzugsweise 0,5 bis 7,5 Gew.-%, Asiaticosid;
0,25 bis 22 Gew.-%, vorzugsweise 0,5 bis 11 Gew.-%, Asiatische Säure und/oder Madekassische Säure;
bis zu 0,5% Iod und wahlweise
0,25 bis 12 Gew.-%, vorzugsweise 0,5 bis 6 Gew.-%, Flavonolglucoside und
0,1 bis 2 Gew.-%, vorzugsweise 0,2 bis 1 Gew.-%, Lactonterpene.

9. Zusammensetzung nach Anspruch 8, **dadurch gekennzeichnet, daß** sie auch eine oder mehrere der folgenden Komponenten enthält:
0,05 bis 2,5 Gew.-%, vorzugsweise 0,1 bis 1 Gew.-%, Coffein;
0,05 bis 4,8 Gew.-%, vorzugsweise 0,1 bis 2,5 Gew.-%, Caffeoylchinasäuren;
0,75 bis 24 Gew.-%, vorzugsweise 1,5 bis 12 Gew.-%, Eicosapentaensäure;
0,6 bis 8 Gew.-%, vorzugsweise 1,2 bis 4 Gew.-%, Docohexaensäure; und
2,5 bis 22 Gew.-%, vorzugsweise 5 bis 11 Gew.-%, Gamma-Linolensäure.

10. Zusammensetzung nach einem der vorhergehenden Ansprüche in einer pharmazeutischen Form zur oralen Verabreichung.

## Revendications

1. Composition à base d'extraits de plantes ayant une activité antioxydante qui est particulièrement utile dans la prévention et le traitement de problèmes circulatoires et dans la prévention et le traitement de l'adiposité, **caractérisée en ce que** ses constituants actifs comprennent en association des biflavones de *Ginkgo biloba*, de la catéchine et/ou de l'épicatéchine, de la coumarine ou des dérivés de celle-ci, de l'iode et un composant choisi parmi l'acide madécassique, l'acide asiatique, l'asiaticoside et des mélanges de ceux-ci.

2. Composition selon la revendication 1, **caractérisée en ce qu'**elle est obtenue en mélangeant des extraits de plantes contenant les principes actifs précités.

3. Composition selon la revendication 2, **caractérisée en ce que** lesdits extraits se présentent sous forme phytosomale.

4. Composition selon l'une quelconque des revendications précédentes, **caractérisée en ce qu'**elle comprend en plus des flavonol glucosides et des terpènes lactoniques.

5. Composition selon l'une quelconque des revendications 1 à 4, **caractérisée en ce qu'**elle comprend également un principe actif choisi dans le groupe constitué par les dérivés de méthylxanthine, les acides chlorogéniques, les acides mono-, et dicaféylquiniques et les dérivés de ceux-ci, l'acide eicosapentaénoïque, l'acide docohexaénoïque, l'acide gamma-linolénique et les mélanges de ceux-ci.

6. Composition selon l'une quelconque des revendications 1 à 4, **caractérisée en ce qu'**elle est obtenue en mélangeant des extraits de plantes selon les pourcentages en poids suivants :
de 1,5 à 32% d'extrait de biflavone de *Ginkgo biloba* ;
de 6 à 80% de leucocyanidine ;
de 1,5 à 32% d'extrait de *Melilotus* et/ou *Aesculus hyppocastanum* ;
de 1,5 à 32% d'extrait de *Centella* ;
de 1,5 à 85% d'extrait de *Fucus vesiculosus* ; et optionnellement
de 1,5 à 32% d'extrait de *Ginkgo biloba* standardisé contenant des glucosides de flavones et des terpènes lactoniques.

7. Composition selon la revendication 6, **caractérisée en ce que**, par rapport à 100 parties du mélange de base de la revendication 6, elle comprend également un ou plusieurs des composants suivants :
de 1,5 à 32% en poids d'extrait d'*Ilex paraguariensis* ;
de 1,5 à 32% en poids d'extrait d'artichaut ;
de 5 à 80% en poids d'huile de poisson ; et
de 30 à 120% en poids d'huile de bourrache.

8. Composition selon l'une quelconque des revendications précédentes, comprenant :
de 0,12 à 12%, de préférence de 0,5 à 6% en poids de biflavones totales ;
de 0,2 à 12%, de préférence de 0,5 à 8% en poids de catéchine et/ou d'épicatéchine ;
de 0,15 à 7%, de préférence de 0,3 à 3,5% en poids de coumarine et de dérivés de celle-ci ;
de 0,25 à 15%, de préférence de 0,5 à 7,5% en poids d'asiaticoside ;
de 0,25 à 22%, de préférence de 0,5 à 11% en poids d'acide asiatique et/ou d'acide madécassique ;
jusqu'à 0,5% d'iode et, optionnellement,
de 0,25 à 12%, de préférence de 0,5 à 6% en poids de flavonol glucosides et
de 0,1 à 2%, de préférence de 0,2 à 1% en poids de terpènes lactoniques.

9. Composition selon la revendication 8, **caractérisée en ce qu'**elle comprend également un ou plusieurs des composants suivants :
de 0,05 à 2,5%, de préférence de 0,1 à 1% en poids de caféine ;
de 0,05 à 4,8%, de préférence de 0,1 à 2,5% en poids d'acides caféylquiniques ;
de 0,75 à 24%, de préférence de 1,5 à 12% en poids d'acide eicosapentaénoïque ;
de 0,6 à 8%, de préférence de 1,2 à 4% en poids d'acide docohexaénoïque ; et
de 2,5 à 22%, de préférence de 5 à 11% en poids d'acide gamma-linolénique.

10. Composition selon l'une quelconque des revendications précédentes, dans une forme pharmaceutique pour l'administration par voie orale.
